# EUROPEAN PATENT APPLICATION

(11) **EP 4 670 708 A1**
(43) Date of publication of application: **31.12.2025**
(21) Application number: 24759566.3
(22) Date of filing: 06.02.2024
(51) Int. Cl.: A61K 8/63, A61Q 5/02, A61Q 5/12, A61Q 7/00, A61P 17/14

(54) **GINSENOSIDE COMPOSITION, DAILY CHEMICAL, AND USE**

(30) Priority: 23.02.2023 CN 202310168637
(71) Applicant: INCIPIRIT TECH (GUANGZHOU) CO., LTD., Guangzhou, Guangdong 510700 (CN); CBIOMEY (GUANGZHOU) TECHNOLOGY CO., LTD., Guangzhou, Guangdong 510700 (CN)
(72) Inventor: REN, Shaohua, Guangzhou, Guangdong 510700 (CN)
(74) Representative: Metida
(86) International application number: PCT/CN2024/076225
(87) International publication number: WO 2024/174889

(57) **Abstract**

The present disclosure relates to a technology field of daily chemical, and provides a ginsenoside composition acting on hair, the ginsenoside composition includes any one group, or a combination of a number of groups selected from N1, N2, N4, N5, and N6. The ginsenoside composition is capable of achieving optimal anti-hair loss and hair growth-promoting effects at a relatively low dosage, which ultimately manifests as a significant reduction in hair loss or visibly denser hair for users. Use of the ginsenoside composition and daily chemical containing the ginsenoside composition are further provided.

## Description

### FIELD

The present disclosure relates to a technology field of daily chemicals, in particular to a ginsenoside composition, a daily chemical, and a use.

### BACKGROUND

Ginseng extract is widely recognized as an effective ingredient for anti-hair loss and hair growth promotion, in Japanese hair regrowth quasi-drug, the ginseng extract is explicitly listed as an effective ingredient for hair growth; relevant patent literatures include that:

CN115154387A relates a method for preparing hair care essence, specifically hair growth-promoting essence and preparation method; the formulation features mild compatibility, comprehensive efficacy, and maximal retention of bioactive components from plant extracts and proteins, the design philosophy focuses on enhancing hair shaft and follicle nutrition, improving follicular skin differentiation, highlighting anti-hair loss efficacy while overcoming limitations of prior art; the hair care essence contains the following compositions: glycerin glucoside, dihydroquercetin glucoside, methylpropanediol, hydrolyzed soy protein, hydrolyzed wheat protein, panthenol, tocopheryl acetate, adenosine, ginseng extract, peony root extract, larch extract, sodium hyaluronate, and deionized water; the mass percentage of each composition includes 6-8.5% of glycerin glucoside, 4-9.5% of dihydroquercetin glucoside, 4-8% of methylpropanediol, 0.5-3% of hydrolyzed soy protein, 0.5-3% of hydrolyzed wheat protein, 0.2-1% of panthenol, 0.2-1% tocopheryl acetate, 0.2-1% of adenosine, 0.3-1% of ginseng extract, 0.3-1% of peony root extract.

CN113577003A relates to a triple-action hair regrowth shampoo including core components with black sesame, panax notoginseng, rose, cnidium monnieri, fennel, cherry, astragalus, jasmine, ginger, polygonum multiflorum, ginseng, angelica, soapberry, black rice, motherwort, polyporus umbellatus, and ligusticum chuanxiong, and including auxiliary components. A preparation method of the triple-action hair regrowth shampoo is further provided. The beneficial effects include activating hair follicles, supplying growth nutrients for hair regrowth, reducing dryness, yellowing, splitting ends of the hair, eliminating dandruff and greasiness, and maintaining synergistic efficacy without mutual inhibition during sustained use

CN113197911A provides a use of a ginsenoside composition in preparing pharmaceuticals for preventing hair loss by acting on follicular tissue. The ginsenoside composition includes ginsenoside Rg1, ginsenoside Re, and ginsenoside CK, the ginsenoside Rg1 and the ginsenoside Re are main active ingredient, the ginsenoside CK is auxiliary ingredient. A use of the ginsenoside composition in preparing pharmaceuticals for enhancing activity of hair follicle tissues, applying or spraying the pharmaceuticals directly onto the scalp to promote the resting hair follicles to enter the regenerative cycle and facilitate hair growth. A use of the ginsenoside composition in preparing pharmaceuticals cultured in vitro for enhancing activity of hair follicle tissues, adding the ginsenoside composition to tissue culture medium for in vitro cultured hair follicle tissues enhances the viability of cultured follicles and significantly improves the survival rate of transplanted hair follicles.

CN112546056A discloses a composition for treating chemotherapy-induced peripheral neuropathy and a use, which relates to a field of medicine. The composition includes ginsenoside Rg1 and fucoidan. The composition can inhibit expression of pro-inflammatory factors such as TNF-α, IL-1β, IL-6, exert anti-inflammatory and restorative effects on peripheral nerves, and enhance resistance. The composition can improve microcirculation disorders, upregulate expression of scavenger receptor A (SR-A), to enhance the phagocytic and clearance ability of macrophages for HMGB1, inhibit overexpression of tissue factor (TF), the multiple target point mechanism mitigates chemotherapy-induced microcirculation dysfunction and neuropathic pain, fundamentally inhibits the development of CIPN (Chemotherapy-Induced Peripheral Neuropathy), thereby achieving a therapeutic goal of efficacy and safety, and providing a new clinical treatment strategy.

After research, it is found that, different ginsenoside combinations (including but is not limited to prototype ginsenoside, protopanaxadiol-type ginsenoside, protopanaxatriol-type ginsenoside, protopanaxadiol secondary ginsenoside, protopanaxatriol secondary ginsenoside) exhibit different effects on proliferation of epidermal keratinocyte and dermal papilla cell, and activity of 5α-reductase and alkaline phosphatase, which means that different combinations have different effects in terms of cytotoxicity, anti-hair loss, hair growth, and hair development.

The technical problem to be solved is that: how to combine different ginsenosides to minimize inactive ingredients while achieving optimal efficacy for hair loss prevention or hair growth promotion.

### SUMMARY

In view of deficiencies of prior art, a purpose of the present disclosure is to provide a ginsenoside composition that can achieve optimal anti-hair loss or hair growth effect at a relatively low dose, ultimately manifested as a more significant reduction in hair loss count or thicker hair for the users.

At the same time, the present disclosure further provides a use of the ginsenoside composition and a daily chemical using the ginsenoside composition.

For achieving the purpose, the present disclosure adopts the following technical solution: a ginsenoside composition acting on hair comprising any one group, or a combination of a plurality of groups selected from N1, N2, N4, N5, and N6;
the N1 includes following components:
   12-28 wt% of ginsenoside Rg1;
   10-16 wt% of ginsenoside Re;
   12-28 wt% of ginsenoside Rb1;
   13-22 wt% of ginsenoside Rc;
   13-22 wt% of ginsenoside Rb2;
   7-13 wt% of ginsenoside Rd;
the N2 includes following components:
   30-50 wt% of ginsenoside Rg1;
   48-72 wt% of ginsenoside Re;
the N4 includes following components:
   8-16 wt% of 20(S)-ginsenoside Rg2;
   7-15 wt% of 20(R)-ginsenoside Rg2;
   17-25 wt% of 20(S)-ginsenoside Rh1;
   16-23 wt% of 20(R)-ginsenoside Rh1;
   5-12 wt% of ginsenoside Rk3;
   16-26 wt% of ginsenoside Rh4;
the N5 includes following components:
   20-40 wt% of ginsenoside Rk3;
   60-80 wt% of ginsenoside Rh4;
the N6 includes following components:
   16-21 wt% of 20(S)-ginsenoside Rg3;
   32-50 wt% of 20(R)-ginsenoside Rg3;
   4-8 wt% of ginsenoside Rk1;
   8-15 wt% of ginsenoside Rg5;
   3-7 wt% of 20(S)-ginsenoside Rh2;
   11-19 wt% of 20(R)-ginsenoside Rh2;
   0.1-1 wt% of ginsenoside Rk2;
   0.5-1.5 wt% of ginsenoside Rh3.

In the ginsenoside composition acting on hair, the N1 includes following components:
18-22 wt% of ginsenoside Rg1;
12-15 wt% of ginsenoside Re;
18-22 wt% of ginsenoside Rb1;
15-19 wt% of ginsenoside Rc;
16-20 wt% of ginsenoside Rb2;
9-11 wt% of ginsenoside Rd;
the N2 includes following components:
   35-45 wt% of ginsenoside Rg1;
   55-65 wt% of ginsenoside Re;
the N4 includes following components:
   10-13 wt% of 20(S)-ginsenoside Rg2;
   8-13 wt% of 20(R)-ginsenoside Rg2;
   18-23 wt% of 20(S)-ginsenoside Rh1;
   17-21 wt% of 20(R)-ginsenoside Rh1;
   6-10 wt% of ginsenoside Rk3;
   18-22 wt% of ginsenoside Rh4;
the N5 includes following components:
   25-35 wt% of ginsenoside Rk3;
   65-75 wt% of ginsenoside Rh4;
the N6 includes following components:
   18-20 wt% of 20(S)-ginsenoside Rg3;
   37-43 wt% of 20(R)-ginsenoside Rg3;
   5-7 wt% of ginsenoside Rk1;
   9-13 wt% of ginsenoside Rg5;
   4-6 wt% of 20(S)-ginsenoside Rh2;
   13-17 wt% of 20(R)-ginsenoside Rh2;
   0.2-0.8 wt% of ginsenoside Rk2;
   0.7-1.2 wt% of ginsenoside Rh3.

In the ginsenoside composition acting on hair, the ginsenoside composition includes at least one of N1, N2, N4, and at least one of N4, N5, N6.

Preferably, in the ginsenoside composition acting on hair, the ginsenoside composition includes at least one of 0.1-99.9wt% of N1, N2, N4, and at least one of 0.1-99.9wt% of N4, N5, N6.

Preferably, in the ginsenoside composition acting on hair, the ginsenoside composition includes at least one of 1-99wt% of N1, N2, N4, and at least one of 1-99wt% of N4, N5, N6.

Preferably, in the ginsenoside composition acting on hair, the ginsenoside composition includes at least one of 10-90wt% of N1, N2, N4, and at least one of 10-90wt% of N4, N5, N6.

Preferably, in the ginsenoside composition acting on hair, the ginsenoside composition includes at least one of 20-80wt% of N1, N2, N4, and at least one of 20-80wt% of N4, N5, N6.

Preferably, in the ginsenoside composition acting on hair, the ginsenoside composition includes at least one of 30-70wt% of N1, N2, N4, and at least one of 30-70wt% of N4, N5, N6.

Preferably, in the ginsenoside composition acting on hair, the ginsenoside composition includes at least one of 40-60wt% of N1, N2, N4, and at least one of 40-60wt% of N4, N5, N6.

Preferably, in the ginsenoside composition acting on hair, the ginsenoside composition includes at least one of 50wt% of N1, N2, N4, and at least one of 50wt% of N4, N5, N6.

At the same time, the present disclosure further provides a daily chemical acting on hair, the daily chemical includes the ginsenoside composition;

a concentration of the ginsenoside composition is 0.05-10000 µg/mL; preferably, the concentration of the ginsenoside composition is 1-5000 µg/mL.

Preferably, in the daily chemical, the concentration of the ginsenoside composition is 10-10000 µg/mL.

It should be noted that, although the concentration in the cell experiments in the present disclosure is relatively low, when applied to daily formulas, it should be 10 times or 100 times higher than the cell concentration.

In the daily chemical acting on hair, the daily chemical is shampoo, a hair conditioner, lyophilized powder, an essence, hair spray, hair cream, or hair lotion.

At the same time, the present disclosure further provides a use of a ginsenoside composition as an anti-hair loss active ingredient in daily chemical, the ginsenoside composition is N1, N2, or N4;
the N1 includes following components:
   12-28 wt% of ginsenoside Rg1;
   10-16 wt% of ginsenoside Re;
   12-28 wt% of ginsenoside Rb1;
   13-22 wt% of ginsenoside Rc;
   13-22 wt% of ginsenoside Rb2;
   7-13 wt% of ginsenoside Rd;
the N2 includes following components:
   30-50 wt% of ginsenoside Rg1;
   48-72 wt% of ginsenoside Re;
the N4 includes following components:
   8-16 wt% of 20(S)-ginsenoside Rg2;
   7-15 wt% of 20(R)-ginsenoside Rg2;
   17-25 wt% of 20(S)-ginsenoside Rh1;
   16-23 wt% of 20(R)-ginsenoside Rh1;
   5-12 wt% of ginsenoside Rk3;
   16-26 wt% of ginsenoside Rh4.

Preferably, the N1 includes following components:
18-22 wt% of ginsenoside Rg1;
12-15 wt% of ginsenoside Re;
18-22 wt% of ginsenoside Rb1;
15-19 wt% of ginsenoside Rc;
16-20 wt% of ginsenoside Rb2;
9-11 wt% of ginsenoside Rd;
the N2 includes following components:
   35-45 wt% of ginsenoside Rg1;
   55-65 wt% of ginsenoside Re;
the N4 includes following components:
   10-13 wt% of 20(S)-ginsenoside Rg2;
   8-13 wt% of 20(R)-ginsenoside Rg2;
   18-23 wt% of 20(S)-ginsenoside Rh1;
   17-21 wt% of 20(R)-ginsenoside Rh1;
   6-10 wt% of ginsenoside Rk3;
   18-22 wt% of ginsenoside Rh4.

At the same time, the present disclosure further provides a use of a ginsenoside composition as an anti-hair loss active ingredient in daily chemical, the ginsenoside composition is N4, N5, or N6;
the N4 includes following components:
   8-16 wt% of 20(S)-ginsenoside Rg2;
   7-15 wt% of 20(R)-ginsenoside Rg2;
   17-25 wt% of 20(S)-ginsenoside Rh1;
   16-23 wt% of 20(R)-ginsenoside Rh1;
   5-12 wt% of ginsenoside Rk3;
   16-26 wt% of ginsenoside Rh4;
the N5 includes following components:
   20-40 wt% of ginsenoside Rk3;
   60-80 wt% of ginsenoside Rh4;
the N6 includes following components:
   16-21 wt% of 20(S)-ginsenoside Rg3;
   32-50 wt% of 20(R)-ginsenoside Rg3;
   4-8 wt% of ginsenoside Rk1;
   8-15 wt% of ginsenoside Rg5;
   3-7 wt% of 20(S)-ginsenoside Rh2;
   11-19 wt% of 20(R)-ginsenoside Rh2;
   0.1-1 wt% of ginsenoside Rk2;
   0.5-1.5 wt% of ginsenoside Rh3.

Preferably, the N4 includes following components:
10-13 wt% of 20(S)-ginsenoside Rg2;
8-13 wt% of 20(R)-ginsenoside Rg2;
18-23 wt% of 20(S)-ginsenoside Rh1;
17-21 wt% of 20(R)-ginsenoside Rh1;
6-10 wt% of ginsenoside Rk3;
18-22 wt% of ginsenoside Rh4;
the N5 includes following components:
   25-35 wt% of ginsenoside Rk3;
   65-75 wt% of ginsenoside Rh4;
the N6 includes following components:
   18-20 wt% of 20(S)-ginsenoside Rg3;
   37-43 wt% of 20(R)-ginsenoside Rg3;
   5-7 wt% of ginsenoside Rk1;
   9-13 wt% of ginsenoside Rg5;
   4-6 wt% of 20(S)-ginsenoside Rh2;
   13-17 wt% of 20(R)-ginsenoside Rh2;
   0.2-0.8 wt% of ginsenoside Rk2;
   0.7-1.2 wt% of ginsenoside Rh3;
   A sum of each component of N1, N2, N4, N5, N6, and N7 is 100%.

Compared with the prior art, the present disclosure has the following beneficial effects:
the present disclosure conducted experiments on the proliferation effects of various combinations of epidermal keratinocytes and dermal papilla cells, the activity of 5α-reductase, and the activity of alkaline phosphatase, it is found that:
the cytotoxicity of the ginsenoside composition N5, the ginsenoside composition N6, and the ginsenoside composition N7 is stronger than that of other ginseng extracts;
the ginsenoside composition N4 has a promoting effect on the proliferation of epidermal keratinocytes, and has a promoting effect on the proliferation of epidermal keratinocytes when tested with a double-layer culture system of dermal papilla cells and epidermal keratinocytes;
the ginsenoside composition N1, the ginsenoside composition N2, and the ginsenoside composition N4 have an effect of inhibiting the activity of 5α-reductase;
the ginsenoside composition N4, the ginsenoside composition N5, and the ginsenoside composition N6 have a promoting effect on the activity of alkaline phosphatase in the cultured dermal papilla cells;
the above means that ginsenoside composition N1, the ginsenoside composition N2, and the ginsenoside composition N4 are effective in preventing male hair loss, while the ginsenoside composition N4, the ginsenoside composition N5, and the ginsenoside composition N6 have hair growth effects;

Overall, it is believed that the ginsenoside composition N4 has performed the best in most tests and is the most suitable choice to be added to daily chemical products for hair loss prevention and hair growth.

### BRIEF DESCRIPTION OF THE DRAWINGS

FIG. 1 is a schematic diagram illustrating effects of a ginsenoside composition on proliferation of epidermal keratinocytes;
   Data presented as mean value+ standard deviation n=3; + represents P<0.1, * represents P<0.05, ** represents P<0.01 vs Control; the same below;
FIG. 2 is a schematic diagram illustrating effects of the ginsenoside composition on proliferation of dermal papilla cells;
FIG. 3 is a schematic diagram illustrating effects of the ginsenoside compositions with a concentration of 2µg/mL on proliferation of epidermal keratinocytes in a co-culture system of the epidermal keratinocytes and the dermal papilla cells;
FIG. 4 is a schematic diagram illustrating effects of the ginsenoside compositions with a concentration of 20µg/mL on proliferation of epidermal keratinocytes in a co-culture system of the epidermal keratinocytes and the dermal papilla cells;
FIG. 5 is a schematic diagram illustrating effects of minoxidil sulfate at various concentrations on proliferation of epidermal keratinocytes in a co-culture system of the epidermal keratinocytes and the dermal papilla cells;
FIG. 6 is a schematic diagram illustrating effects of the ginsenoside compositions on activity of 5α-reductase;
FIG. 7 is a schematic diagram illustrating effects of the ginsenoside composition of group N4 on the activity of 5α-reductase;
FIG. 8 is a schematic diagram illustrating activity staining of alkaline phosphatase of the cultured dermal papilla cells treated with the ginsenoside composition with a concentration of 2µg/mL;
FIG. 9 is a schematic diagram illustrating activity staining of alkaline phosphatase of the cultured dermal papilla cells treated with the ginsenoside composition with a concentration of 20µg/mL;
FIG. 10 is a schematic diagram illustrating effects of the ginsenoside composition on the activity of alkaline phosphatase of the cultured dermal papilla cells;
FIG. 11 is a schematic diagram illustrating effects of valproic acid as a positive control at various concentrations on the activity of alkaline phosphatase of the cultured dermal papilla cells;
FIG. 12 is a testing conclusion for report No. G522-2210027;
FIG. 13 is a testing conclusion for report No. G522-2209047;
FIG. 14 is a microscopic image of scalp of selected human trial participants;
FIG. 15 is a hair image of selected human trial participants.

### DETAILED DESCRIPTION

The following further illustrates the technical solutions of the present disclosure through specific implementation methods. Those skilled in the art should understand that the embodiments are merely to aid in understanding the present disclosure and should not be construed as specific limitations.

The experimental steps or conditions not specified in the embodiments, can be performed according to the conventional experimental steps or conditions described in the literature in the art. Reagents or instruments not indicating the manufacturer are conventional reagent products that can be obtained commercially.

### Embodiment 1

A ginsenoside composition, containing the components shown in Table 1 below:

**Table 1 Ginsenoside formula table (unit wt%)**

| | N1 | N1-2 | N1-3 | N1-4 | N1-5 |
|---|---|---|---|---|---|
| Ginsenoside Rg1 | 20 | 17 | 24 | 18 | 22 |
| Ginsenoside Re | 14 | 15 | 10 | 15 | 12 |
| Ginsenoside Rb1 | 20 | 17 | 25 | 22 | 22 |
| Ginsenoside Rc | 18 | 21 | 13 | 15 | 15 |
| Ginsenoside Rb2 | 18 | 17 | 20 | 20 | 20 |
| Ginsenoside Rd | 10 | 13 | 8 | 10 | 9 |
| Total | 100 | 100 | 100 | 100 | 100 |

### Embodiment 2

A ginsenoside composition, containing the components shown in Table 2 below:

**Table 2 Ginsenoside formula table (unit wt%)**

| | N2 | N2-2 | N2-3 | N2-4 | N2-5 |
|---|---|---|---|---|---|
| Ginsenoside Rg1 | 40 | 30 | 50 | 35 | 45 |
| Ginsenoside Re | 60 | 70 | 50 | 65 | 55 |
| Total | 100 | 100 | 100 | 100 | 100 |

### Embodiment 3

A ginsenoside composition (N3), containing the following components:
Ginsenoside Rb130 wt%;
Ginsenoside Rc 26 wt%;
Ginsenoside Rb2 27 wt%;
Ginsenoside Rd 17 wt%.

### Embodiment 4

A ginsenoside composition, containing the components shown in Table 3 below:

**Table 3 Ginsenoside formula table (unit wt%)**

| | N4 | N4-1 | N4-2 | N4-3 | N4-4 | N4-5 |
|---|---|---|---|---|---|---|
| 20(S)-Ginsenoside Rh1 | 22 | 23 | 19 | 25 | 23 | 21 |
| 20(S)-Ginsenoside Rg2 | 14 | 15 | 10 | 16 | 13 | 13 |
| 20(R)-Ginsenoside Rg2 | 11 | 15 | 15 | 9 | 13 | 13 |
| 20(R)-Ginsenoside Rh1 | 21 | 22 | 23 | 19 | 19 | 21 |
| Ginsenoside Rk3 | 10 | 9 | 7 | 12 | 10 | 10 |
| Ginsenoside Rh4 | 22 | 16 | 26 | 19 | 22 | 22 |
| Total | 100 | 100 | 100 | 100 | 100 | 100 |

### Embodiment 5

A ginsenoside composition, containing the components shown in Table 4 below:

**Table 4 Ginsenoside formula table (unit wt%)**

| | N5 | N5-2 | N5-3 | N5-4 | N5-5 |
|---|---|---|---|---|---|
| Ginsenoside Rk3 | 29 | 20 | 40 | 25 | 35 |
| Ginsenoside Rh4 | 71 | 80 | 60 | 75 | 65 |
| Total | 100 | 100 | 100 | 100 | 100 |

### Embodiment 6

A ginsenoside composition, containing the components shown in Table 5 below:

**Table 5 Ginsenoside formula table (unit wt%)**

| | N6 | N6-2 | N6-3 | N6-4 | N6-5 |
|---|---|---|---|---|---|
| 20(S)- Ginsenoside Rg3 | 19 | 16 | 21 | 18 | 20 |
| 20(R)- Ginsenoside Rg3 | 40.4 | 45.5 | 42.5 | 41.5 | 41 |
| Ginsenoside Rk1 | 7 | 4 | 8 | 5 | 7 |

| | | | | | |
|---|---|---|---|---|---|
| Ginsenoside Rg5 | 11 | 13 | 9 | 13 | 11 |
| 20(S)- Ginsenoside Rh2 | 5 | 3 | 7 | 4 | 6.5 |
| 20(R)- Ginsenoside Rh2 | 16 | 17 | 11 | 17 | 13 |
| Ginsenoside Rk2 | 0.6 | 0.1 | 1 | 0.5 | 0.5 |
| Ginsenoside Rh3 | 1 | 1.4 | 0.5 | 1 | 1 |
| Total | 100 | 100 | 100 | 100 | 100 |

### Embodiment 7

A ginsenoside composition, containing the following components:
Ginsenoside Rk1 13 wt%;
20(S)-Ginsenoside Rh2 23 wt%;
20(R)-Ginsenoside Rh2 13 wt%;
Ginsenoside Rk2 20 wt%;
Ginsenoside Rh3 31 wt%.

The preparation method for Embodiments 1-7:
Precisely weigh each component and mix them together.

### Performance Test

Dissolve the ginsenoside compositions N1, N2, N3, N4, N5, N6, and N7 in dimethyl sulfoxide (DMSO), to prepare samples with concentrations of 20 mg/ml, 2 mg/ml, 0.2 mg/ml, and 0.02 mg/ml, respectively.

In addition, minoxidil sulfate (Sigma-Aldrich) was dissolved in DMSO to prepare solutions with concentrations of 2 mg/ml, 0.2 mg/ml, 0.02 mg/ml, and 0.002 mg/ml as positive controls.

At the same time, finasteride (Sigma-Aldrich) was also dissolved in DMSO to prepare a solution with a concentration of 1.5 µg/ml (4 µM).

### 1. Effects of ginseng extracts on the proliferation of epidermal keratinocytes and dermal papilla cells

A solution of human epidermal keratinocytes at a concentration of 200,000 cells/cell was inoculated into a 96-well cell culture plate at 100µl/well (n=3), and cultured for one day. Then, 1 µl/well of ginseng extracts 1-7, the positive control (Minoxidil sulfate), and a blank control (DMSO) were added, followed by continued culture for 3 days. Subsequently, 10 µl/well of Cell Counting Kit-8 (Dojindo Molecular Technologies, Inc.) was added and cultured for a further 2 hours, cell counting was performed using a microplate reader (450 nm, Mix 0).

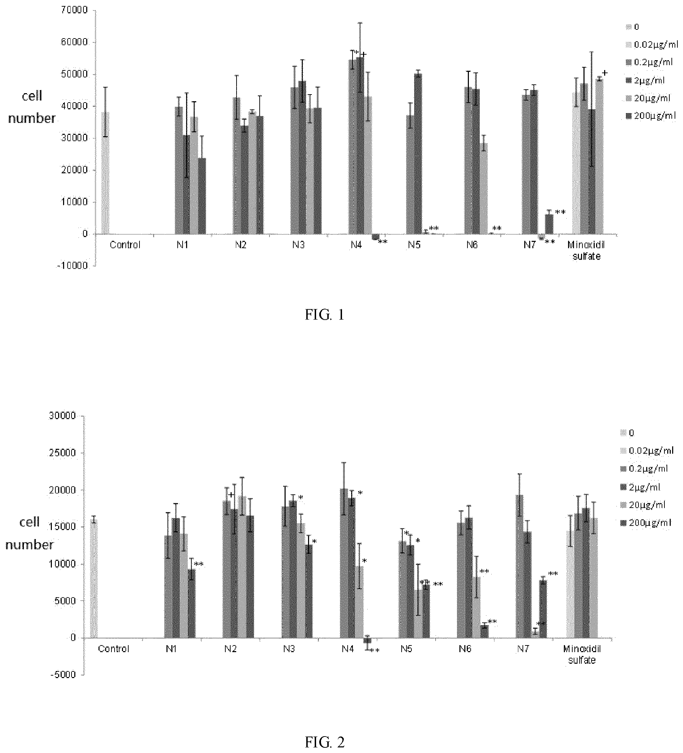

The effects of the ginsenoside composition on the proliferation of the epidermal keratinocytes can be referred to in FIG. 1.

In FIG. 1, the ginsenoside composition N4 significantly increased the cell number at concentrations of 0.2 µg/ml and 2 µg/ml (p values P<0.05 and P<0.1, respectively).

The effects was stronger than that of the positive control Minoxidil sulfate (0.02 µg/ml, 0.2 µg/ml, 2 µg/ml, 20 µg/ml). At the same time, the ginsenoside compositions N4, N5, N6, and N7 significantly reduced the cell number at the concentration of 200 µg/ml. The ginsenoside composition N1 also reduced the cell number at the concentration of 200 µg/ml.

A solution of human dermal papilla cells at a concentration of 100,000 cells/cell was inoculated into the 96-well cell culture plate at 100 µl/well (n=3), and cultured for one day. Proliferation and counting of dermal papilla cells were performed similarly. The concentration of test samples ranged from 0.2 µg/ml to 200 µg/ml, and the concentration of minoxidil sulfate ranged from 0.02 µg/ml to 20 µg/ml.

Referring to FIG. 2, FIG. 2 illustrates the effects of the ginsenoside composition on the proliferation of the dermal papilla cells.

The ginsenoside composition N4 had an effect of increasing cell number at a concentration of 2 µg/ml (P<0.05).

The effect was stronger than that of the positive control minoxidil sulfate (0.02 µg/ml, 0.2 µg/ml, 2 µg/ml, 20 µg/ml). The ginsenoside composition N3 also had an effect of increasing cell number at the concentration of 2 µg/ml (P<0.05). The ginsenoside composition N2 showed a tendency to increase cell number at the concentration of 0.2 µg/ml (P<0.1). The ginsenoside compositions N4 and N6 significantly reduced cell number at the concentration of 200 µg/ml. The ginsenoside compositions N1, N3, N5, and N7 also reduced cell number at the concentration of 200 µg/ml.

The method utilizes the principle that when the water-soluble tetrazolium salt generated by the highly sensitive water-soluble yellow formazan product is reduced by dehydrogenases in viable cells, the absorbance of the generated water-soluble yellow formazan product was measured at 450nm to calculate the number of viable cells. The cell number has a linear relationship with the amount of formazan.

### 2. Effects of ginseng extract on the proliferation of epidermal keratinocytes in a co-culture system of dermal papilla cells and epidermal keratinocytes

Add 500 µl of human epidermal keratinocytes (30,000 cells/ml) to each well of a 24-well MULTIWELL plate, then add a 1.0 µm of cell culture insert, and culture for 1 day. The 1.0 µm of cell culture insert already contains 300 µl of dermal papilla cells (20,000 cells/ml). Subsequently, add 1 µl/well each of the ginseng extracts 1-7, the positive control minoxidil sulfate, and the blank control (DMSO), and continue culturing for 3 days. Remove the 1.0 µm of cell culture insert, add 50 µl/well of Cell Counting Kit-8 (Dojindo Molecular Technologies, Inc.), continue culturing for 2 hours, and determine cell number using a microplate reader (450 nm, Mix 0).

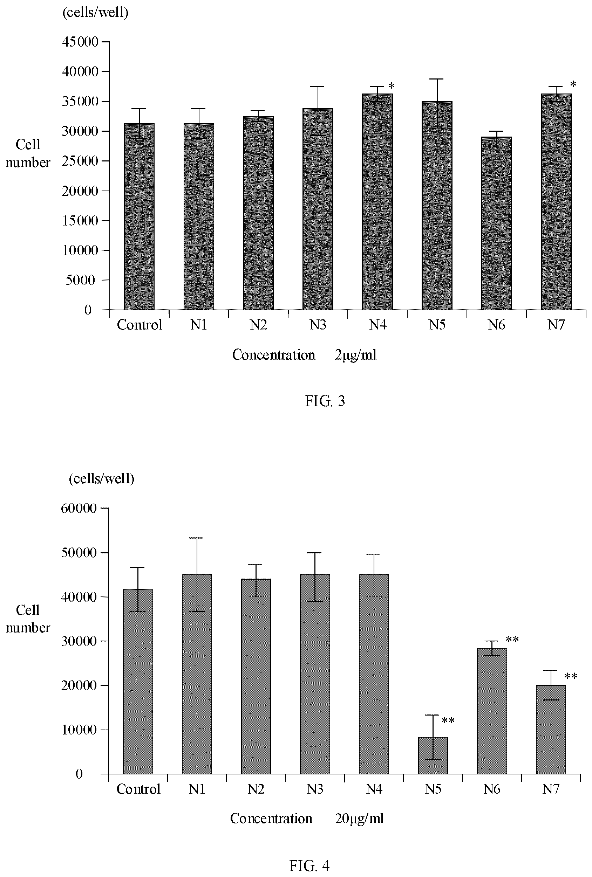

Referring to FIG. 3, FIG. 3 illustrates the effects of the ginseng extract on the proliferation of the epidermal keratinocytes in the co-culture system of dermal papilla cells and keratinocytes at a ginsenoside composition concentration of 2 µg/ml.

Referring to FIG. 4, FIG. 4 illustrates the effects of the ginseng extract on the proliferation of the epidermal keratinocytes in the co-culture system of dermal papilla cells and keratinocytes at a ginsenoside composition concentration of 20 µg/ml.

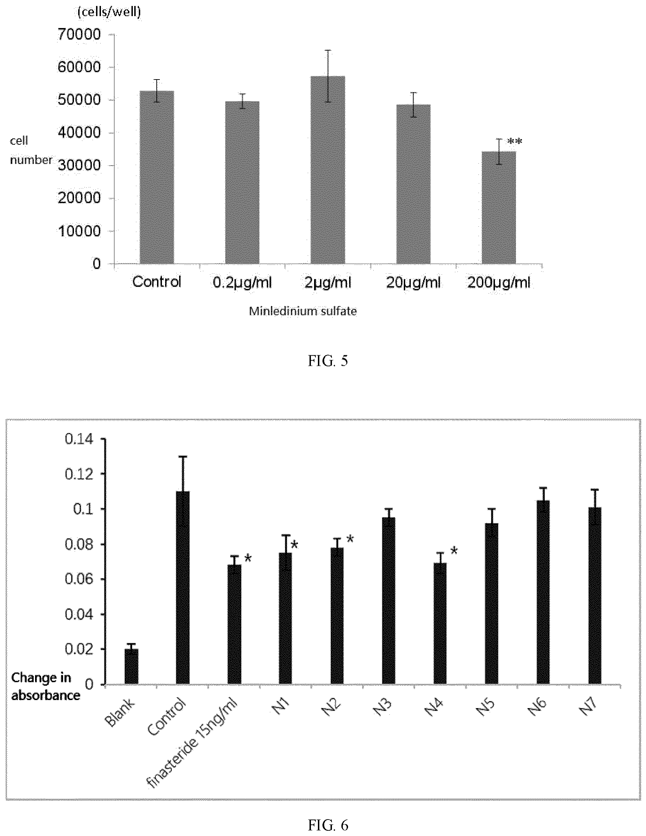

Referring to FIG. 5, FIG. 5 illustrates the effects of minoxidil sulfate at different concentrations on the proliferation of the epidermal keratinocytes in the co-culture system of dermal papilla cells and keratinocytes.

It is found that, in the experiment using the dermal papilla cell and keratinocyte co-culture system, the ginsenoside compositions N4 and N7 promoted the proliferation of keratinocytes at the concentration of 2 µg/ml. The ginsenoside compositions N5, N6, N7 had inhibitory or cytotoxic effects at the concentration of 20 µg/ml. The positive control minoxidil sulfate (0.2 µg/ml, 2 µg/ml, 20 µg/ml, 200 µg/ml) showed no effect in promoting the proliferation of keratinocyte.

### 3. Effects of ginseng extracts on activity of 5α-reductase

5α-reductase is the enzyme that promotes the conversion of testosterone to 5α-dihydrotestosterone, this experiment utilized the NAD cycling method to determine the effect of ginseng extracts on activity of 5α-reductase.

Prepare solution A by adding 520 µl of a potassium phosphate buffer (pH 5.0) solution containing 0.3 M sucrose/1 mM dithiothreitol/40 mM, 80 µl of 100 µM testosterone, and 40 µl of 16 µM NADPH to a 1.5 ml tube. Take 83 µl of solution A, add 1 µl of ginseng extract and 16 µl of rat liver microsomes, and culture for 20 minutes. Then heat at 80°C for 5 minutes, add 600 µl of Tris-HCl buffer (pH 9.8) and 40 µl of 20 mM thio-NAD, and continue culturing for 10 minutes. After centrifugation, take 100 µl solution and add the solution to a microplate well, then add 10 µl of 400 U/ml 3α-HSD, and measure absorbance at 400 nm within 30 minutes. Finasteride (40 nM) was used as the positive control.

FIG. 6 is a schematic diagram showing the effects of the ginsenoside compositions on activity of 5α-reductase.

The ginsenoside compositions N1, N2, N4 (200 µg/ml) inhibited activity of 5α-reductase. The strength of inhibition was comparable to that of 15 ng/ml (40 nM) finasteride.

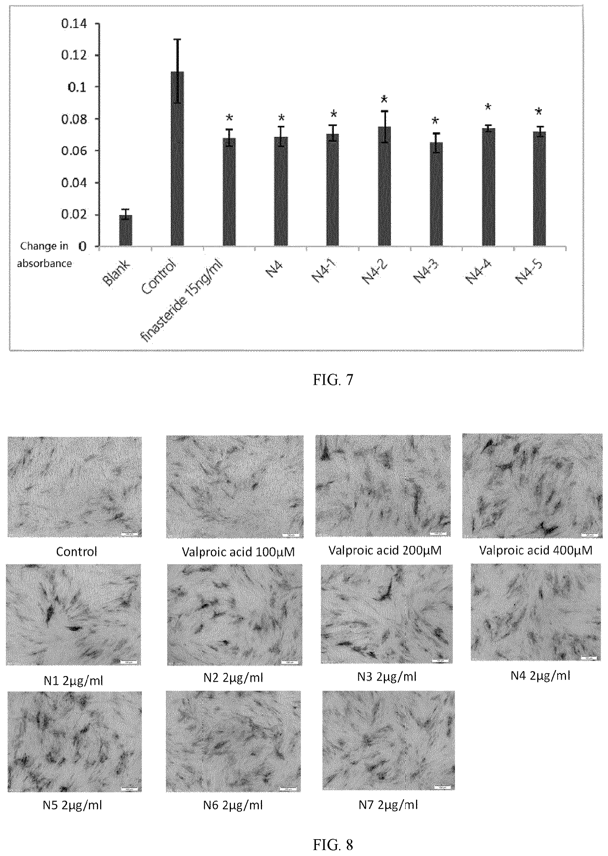

FIG. 7 shows that the ginsenoside compositions N4, N4-1, N4-2, N4-3, N4-4, N4-5 (200 µg/ml) inhibited activity of 5α-reductase. The strength of inhibition was almost equivalent.

### 4. Effects of ginseng extracts on activity of alkaline phosphatase

Prepare a solution of human dermal papilla cells at a concentration of 100,000 cells/ml, and add 100 µl/well (n=3) to a 96-well cell culture plate, culture for 1 day. Test sample concentrations ranged from 0.2 µg/ml to 200 µg/ml, and minoxidil sulfate concentrations ranged from 0.02 µg/ml to 20 µg/ml. After culturing for 3 days, fix with 4% PFA, wash with PBS, and measure the activity of alkaline phosphatase using BCIP-NBT solution at 405 nm.

FIG. 8 shows activity staining of alkaline phosphatase in cultured dermal papilla cells treated with the ginsenoside compositions at a concentration of 2 µg/ml; valproic acid was used as the positive control.

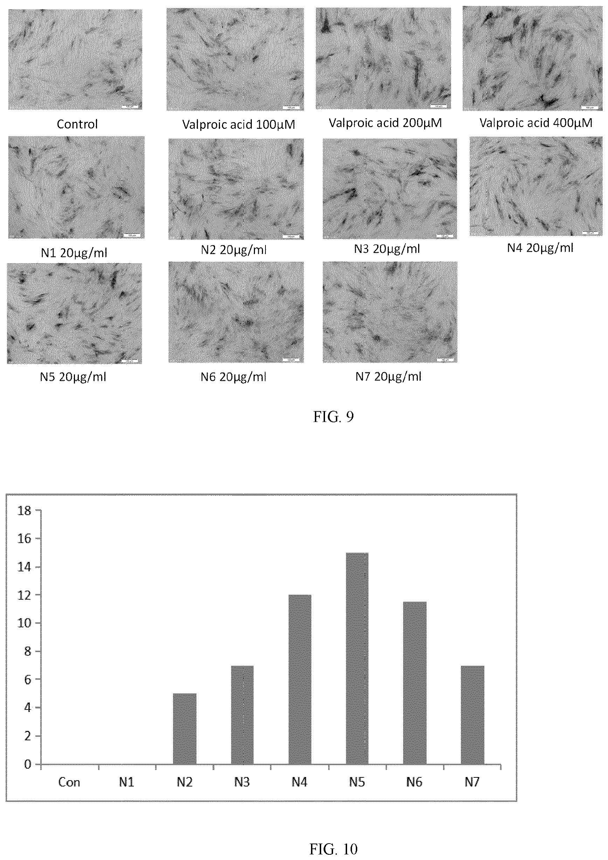

FIG. 9 shows activity staining of alkaline phosphatase in cultured dermal papilla cells treated with the ginsenoside compositions at a concentration of 20 µg/ml; valproic acid was used as the positive control.

FIG. 10 is a schematic diagram showing the effects of the ginsenoside compositions on activity of alkaline phosphatase in cultured dermal papilla cells.

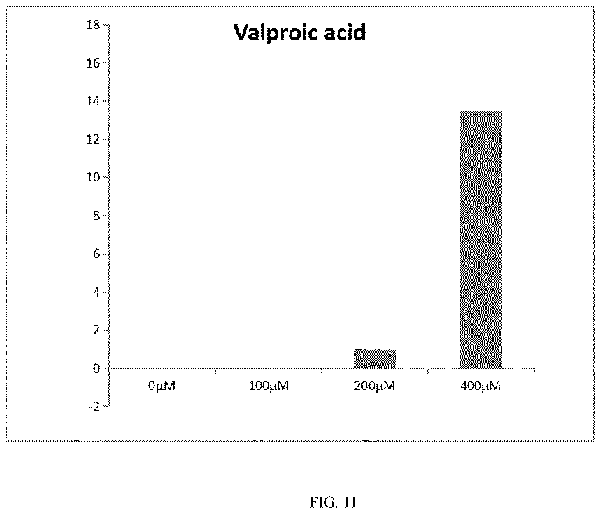

FIG. 11 shows the effects of valproic acid, used as the positive control, at different concentrations on activity of alkaline phosphatase in cultured dermal papilla cells.

### Results analysis:

1. The ginsenoside compositions N5, N6, and N7 exhibited stronger cytotoxicity than the other ginseng extracts (referring to FIG. 4).
2. Referring to FIGS. 1-4, the ginsenoside composition N4 promoted the proliferation of epidermal keratinocytes, this proliferative effect on epidermal keratinocytes was also observed when tested using the dermal papilla cell and epidermal keratinocyte co-culture system.
3. Referring to FIG. 6, the ginsenoside compositions N1, N2, and N4 inhibited activity of 5α-reductase, which indicates that N1, N2, and N4 can significantly prevent male hair loss.
4. Referring to FIG. 7, the ginsenoside compositions N4, N4-1, N4-2, N4-3, N4-4, N4-5 exhibited nearly consistent inhibition of activity of 5α-reductase, which indicates that N4, N4-1, N4-2, N4-3, N4-4, N4-5 can all effectively and significantly prevent male hair loss.
5. Referring to FIGS. 8-10, the ginsenoside compositions N4, N5, and N6 promoted activity of alkaline phosphatase in cultured dermal papilla cells, which indicates that N4, N5, N6 have hair growth-promoting and hair proliferation efficacy.

In summary, N1, N2, and N4 have the efficacy to prevent male hair loss; N4, N5, and N6 have hair growth-promoting and hair proliferation efficacy; N5, N6, and N7 exhibit cytotoxicity.

From the perspectives of promoting epidermal keratinocyte proliferation, preventing hair loss, promoting hair growth, and hair proliferation, N4 is the component with the most promising commercial application potential.

As a more effective approach, it is suggested to combine any one of N1, N2, and N4 with any one of N4, N5, and N6.

More effectively, it is suggested to combine any one of N1, N2 with N4.

The formula of N4 of the present disclosure, under the trade name "ChronoShen^{®} Night SPC", was submitted to Shanxi Boxi General Testing Technology Co., Ltd. for "In Vitro Anti-Hair Loss Efficacy Testing"; N4 is the only ingredient with active efficacy; other components are auxiliary solvents and preservatives without anti-hair loss or hair growth functions. This test was divided into four parts: part 1, based on dermal papilla cells, carries out cytotoxicity detection, to determine the safe administration concentration of the sample on dermal papilla cells; part 2, utilizes dihydrotestosterone (DHT) to stimulate dermal papilla cells, evaluates the anti-hair loss and hair growth efficacy of the test sample by detecting VEGF content and DKK1 gene changes in dermal papilla cells after sample treatment; part 3, based on dermal papilla cells, evaluates the hair growth efficacy of the test sample by detecting cell proliferation after sample treatment; part 4, evaluates the anti-hair loss efficacy of the test sample based on in vitro inhibition of 5α-reductase activity.

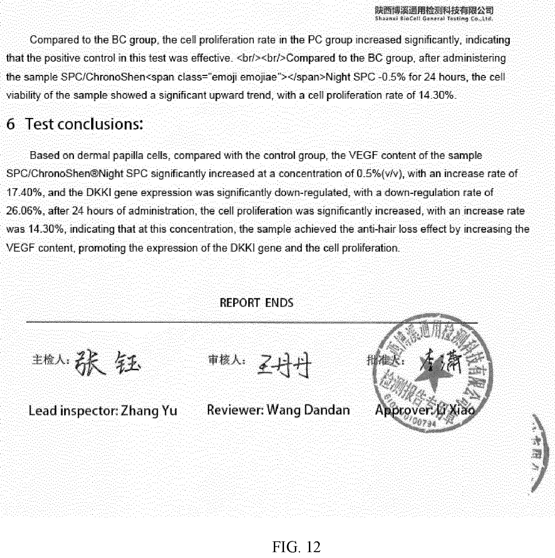

Test report number: G522-2210027; Test date: August 25 - September 25, 2022; Test conclusions can be seen in FIG. 12.

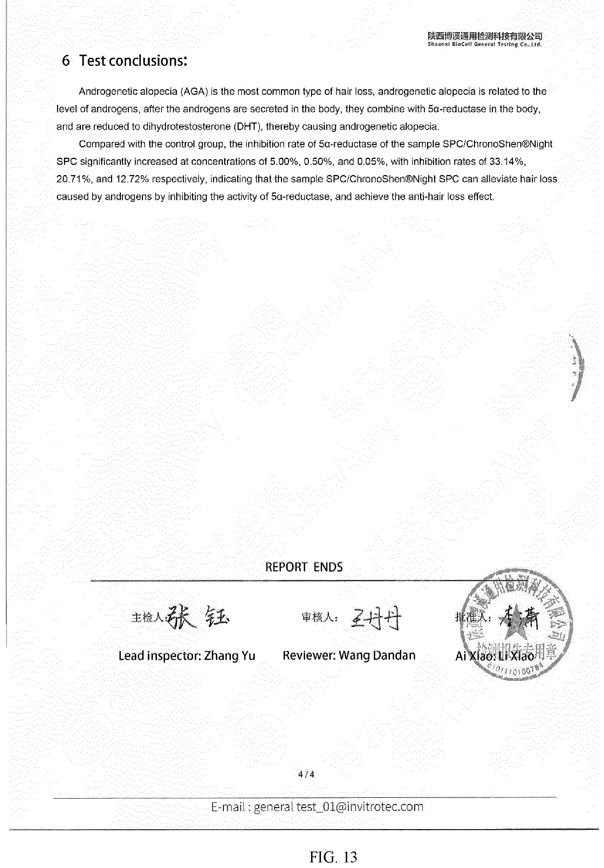

Test report number: G522-2209047; Test date: August 24 - September 4, 2022; Test conclusions can be seen in FIG. 13.

The composition of the raw material "ChronoShen^{®} Night SPC" used in the above two test reports is shown in Table 6 below:

**Table 6 Formula table**

| component | Component content (%) |
|---|---|
| N4 | 1 |
| 1, 3-butanediol | 98 |
| 1, 2-hexanediol | 1 |

The above test results also confirm that the N4 formula has good anti-hair loss and hair growth-promoting effects.

At the same time, the above "ChronoShen^{®} Night SPC" was added to a common essence (see Formula Table 7) to form an anti-hair loss essence, ChronoShen^{®} Night SPC is the only ingredient with active efficacy, and other components are auxiliary solvents and preservatives without anti-hair loss or hair growth functions, the anti-hair loss essence was submitted to CTI Testing Technology Group Co., Ltd. for human testing, sample number HBO00942001, the experiment was divided into hair loss count testing, overall hair density testing, and local skin hair testing.

**Table 7 Formula table of ChronoShen ^{®} Night SPC scalp essence**

| Phase Type | Component | Percentage content(%) | Effect |
|---|---|---|---|
| A | Butanediol | 4.00 | Humectant |
| | Capo 981 | 0.15 | Thickener |
| | Sodium hyaluronate with small molecules | 0.05 | Scalp moisturizing |
| | Water | To 100 | Solvent |
| B | Sodium hydroxide | 0.012 | pH Regulator |
| C | Ethyl alcohol | 8.00 | Solvent |
| | Menthol | 0.04 | Cool and relieve itching |
| | Borneol | 0.06 | Cool and relieve itching |
| D | ChronoShen^{®} Night SPC(water, butanediol, N4, 1, 2-hexanediol) | 2.00 | Anti-aging for the scalp, soothing, oil control, anti-hair loss |
| | glycerol caprylate, Octyl hydroxamic acid | 0.80 | Preservative |
| | 1, 2-hexanediol | 1.00 | Anti-corrosion efficiency enhancement |

Process:
add water to the water pot, slowly sprinkle in carbomer, start stirring, heat to 85°C;
transfer the water from the water pot to the main pot, add the pre-mix of butylene glycol and sodium hyaluronate, keep at 85°c, stir until uniform;
cool down to 60°C, add the aqueous solution of phase B, stir until uniform;
cool down to 40°C, add the pre-mix of phase C, stir until uniform; then sequentially add phase D, stir until uniform;
take a sample for testing, pH 5.5-6.5, if qualified, discharge the batch.

Physicochemical indicators:
appearance: transparent essence-like liquid
pH: 5.5-6.5
hair loss count test results see table 8;

**Table 8 hair loss count test results**

| Descriptive statistics | Head | | | |
|---|---|---|---|---|
| | D0 | D28 | D56 | D84 |
| Number of people | 10 | 10 | 10 | 10 |
| Mean value | 21 | 19 | 13 | 7 |
| Standard deviation | 7 | 11 | 11 | 5 |
| Maximum value | 32 | 42 | 40 | 18 |
| Minimum value | 11 | 4 | 5 | 0 |
| Mid-value | 20 | 17 | 9 | 7 |

From the above test, it can be found that after 10 subjects used the sample for 28 days, the hair loss count decreased by 9.52%; after 56 days of use, the hair loss count decreased by 38.10%; after 84 days of use, the hair loss count decreased by 66.67%. During the product use period, as the usage time extended, the hair loss condition of the subjects significantly improved.

Overall hair density test results see table 9;

**Table 9 Overall hair density statistics**

| Descriptive statistics | Head | | | |
|---|---|---|---|---|
| | D0 | D28 | D56 | D84 |
| Number of people | 10 | 10 | 10 | 10 |
| Mean value | 3.8 | 4.0 | 4.3 | 4.5 |
| Standard deviation | 0.8 | 0.9 | 0.8 | 0.8 |
| Maximum value | 4.8 | 5.0 | 5.0 | 5.0 |
| Minimum value | 2.0 | 2.0 | 2.3 | 2.5 |
| Mid-value | 4.0 | 4.2 | 4.5 | 4.5 |

From the above test, it can be seen that as the usage time increased, after 10 subjects used the sample for 28 days, the overall hair density increased by 5.26%; after 56 days of use, the overall hair density increased by 13.16%; after 84 days of use, the overall hair density increased by 18.42%.

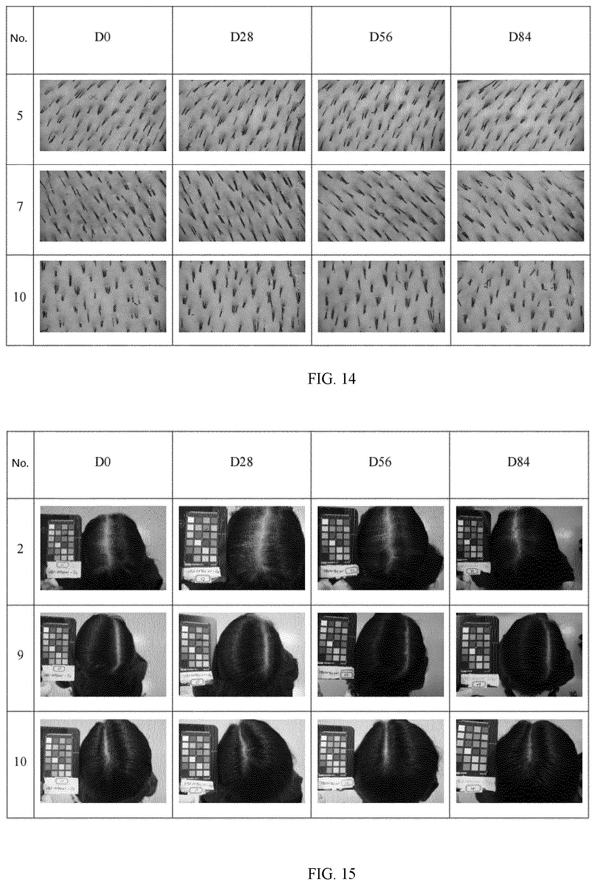

FIG. 14 shows a number of microscope photos of the scalp of some subjects.

FIG. 15 shows a number of hair photos of some subjects.

Through human testing, it can be seen that when the composition N4 of the present disclosure is used as an essence, it can significantly inhibit hair loss and increase hair density.

The following provides formulation examples of the composition (N4) of the present disclosure applied to other representative scalp formulations;

In the tables below, "Ginsenoside" refers to composition (N4). As follows:

**Table 10 Shenan mild anti-hair loss shampoo**

| Phase Type | Product name | Component name | Percentage content(%) |
|---|---|---|---|
| A | Water | Water | |
| | JR400 | Polyquaternium-10 | 0.25 |
| | EDTA-2Na | EDTA-2Na | 0.05 |
| B | CAB35 | Cocamidopropyl betaine | 15.00 |
| | NGS LMA30 | Sodium lauroyl methylaminopropionate | 18.00 |
| | CMT30 | Sodium methyl cocoyl taurate | 15.00 |
| | 120T | PEG-120 Methyl glucose trioleate | 0.30 |
| | 7908 | Glyceryl laurate | 0.50 |
| C | M7 | Water, Polylysine, Polyquaternium-73 | 0.60 |
| | PG100 | Polyglyceryl-10 Dipalmitate, Palmitamide trimethylammonium chloride | 0.80 |
| D | ChronoShen ^{®} Night SPC | Butylene Glycol, Ginsenoside, 1,2-hexanediol | 0.50 |
| | 9010 | Phenoxyethanol, Ethylhexylglycerin | 0.60 |
| E | 6511 | Cocamide MEA | 2.00 |
| | Fragrance | Fragrance | 0.30 |
| | Citric acid (50%) | Citric acid | As needed |

Process:
1. Phase A, B: heat the water temperature to 85°C, add phases A and B, and stir until dissolved.
2. Phase C, D: cool down to 40°C, add phases C and D sequentially, and stir until uniform.
3. Add phase F, adjust the pH, and stir until uniform.
4. Stop stirring, discharge the batch.

Physicochemical indicators:
appearance: transparent and viscous liquid;
Viscosity: (25°C, 3#6rpm) : 5000-12000 MPa.s;
pH (20°C, 10% aqueous solution) : 6.2-6.4.

**Table 11 Shenjing oil-control and anti-hair loss shampoo**

| Phase Type | Product name | Component name | Percentage content(%) |
|---|---|---|---|
| A | AQUA | Water | to 100.00 |
| | AES | Sodium lauryl ether sulfate | 7.00 |
| | NGS PG66 | Alkyl polyglucoside | 9.00 |
| | K12A | Ammonium lauryl sulfate | 2.00 |
| | EDTA-2Na | EDTA-2Na | 0.05 |
| | JR3000 | Polyquaternium-10 | 0.30 |
| B | PG100 | Polyglyceryl-10 dipalmitate, Palmitamide trimethylammonium chloride | 2.00 |
| C | SF_1 | Acrylates copolymer | 5.00 |
| | AQUA | Water | 10.00 |
| D | ChronoShen^{®} Night SPC | Water, Butylene glycol, Ginsenoside, 1,2-Hexanediol | 2.00 |
| | Trehalose | Trehalose | 0.40 |
| | Green particle | Lactose, Cellulose, Hydroxypropyl Methylcellulose, Jojoba lipids | 0.30 |
| E | Liquid caustic soda (32%) | Sodium hydroxide | as needed |
| | CAB35 | Cocamidopropyl betaine | 5.00 |
| | Essence | Essence | 0.10 |
| | LRI | PEG-40 Hydrogenated castor oil, PPG-26 buteth-26 | 0.30 |
| | Phenoxyethanol | Phenoxyethanol | 0.60 |

Process:
1. Phase A: Heat to 85°C, and stir to dissolve.
2. Phases B, C, D and E: add after cooling to 40°C, disperse and dissolve SF-1 in water in advance, then add it to the material, and stir until uniform.
3. Stop stirring, discharge the batch.

Physicochemical indicators:
Appearance: green particle transparent liquid;
Viscosity: (25°C, 3#6rpm) : 9000-12000 MPa.s;
pH (20°C, 10% aqueous solution) : 6.5-6.8.

**Table 12 anti-hair loss freeze-dried powder**

| Serial number | Component name | Percentage content(%) | Effect |
|---|---|---|---|
| 1 | Chrono Shen^{®} Night SPC FD (Ginsenoside, β-Glucan) | 26.50 | Soothing and Repairing, oil control, anti-aging, hair loss prevention |
| 2 | Glyceryl polyether | 73.50 | Moisturizing |

Through the above testing, it is can be seen that:
When different ginsenosides are combined, the rare protopanaxatriol-type ginsenoside secondary glycoside combinations (N4 and N5), the rare protopanaxadiol-type ginsenoside secondary glycoside combinations (N6, N7), the mixed prototype ginsenosides (N1), the protopanaxadiol-type ginsenosides (N2), and the protopanaxatriol-type ginsenosides (N3) exhibit different trends, N4 demonstrated the best effects in multiple tests and is a combination worthy of ongoing research.

The outstanding contributions of the present disclosure include that:
1. Different ginsenosides exhibit different characteristics, during use, the ginsenosides should be flexibly selected and blended according to the characteristics of the product formulation and based on the above experimental results.
2. The present disclosure provides several sets of raw material combinations that are as optimized as possible and used in the least amount to adapt to the formulations of daily chemical products with different functions.

## Claims

1. A ginsenoside composition acting on hair comprising any one group, or a combination of a plurality of groups selected from N1, N2, N4, N5, and N6;
wherein the N1 comprises following components:
12-28 wt% of ginsenoside Rg1;
10-16 wt% of ginsenoside Re;
12-28 wt% of ginsenoside Rb1;
13-22 wt% of ginsenoside Rc;
13-22 wt% of ginsenoside Rb2;
7-13 wt% of ginsenoside Rd;
wherein the N2 comprises following components:
30-50 wt% of ginsenoside Rg1;
48-72 wt% of ginsenoside Re;
wherein the N4 comprises following components:
8-16 wt% of 20(S)-ginsenoside Rg2;
7-15 wt% of 20(R)-ginsenoside Rg2;
17-25 wt% of 20(S)-ginsenoside Rh1;
16-23 wt% of 20(R)-ginsenoside Rh1;
5-12 wt% of ginsenoside Rk3;
16-26 wt% of ginsenoside Rh4;
wherein the N5 comprises following components:
20-40 wt% of ginsenoside Rk3;
60-80 wt% of ginsenoside Rh4;
wherein the N6 comprises following components:
16-21 wt% of 20(S)-ginsenoside Rg3;
32-50 wt% of 20(R)-ginsenoside Rg3;
4-8 wt% of ginsenoside Rk1;
8-15 wt% of ginsenoside Rg5;
3-7 wt% of 20(S)-ginsenoside Rh2;
11-19 wt% of 20(R)-ginsenoside Rh2;
0.1-1 wt% of ginsenoside Rk2;
0.5-1.5 wt% of ginsenoside Rh3;
and a sum of the components of each of N1, N2, N4, N5, N6, and N7 is 100%.

2. The ginsenoside composition acting on hair according to claim 1, wherein the N1 comprises following components:
18-22 wt% of ginsenoside Rg1;
12-15 wt% of ginsenoside Re;
18-22 wt% of ginsenoside Rb1;
15-19 wt% of ginsenoside Rc;
16-20 wt% of ginsenoside Rb2;
9-11 wt% of ginsenoside Rd;
wherein the N2 comprises following components:
35-45 wt% of ginsenoside Rg1;
55-65 wt% of ginsenoside Re;
wherein the N4 comprises following components:
10-13 wt% of 20(S)-ginsenoside Rg2;
8-13 wt% of 20(R)-ginsenoside Rg2;
18-23 wt% of 20(S)-ginsenoside Rh1;
17-21 wt% of 20(R)-ginsenoside Rh1;
6-10 wt% of ginsenoside Rk3;
18-22 wt% of ginsenoside Rh4;
wherein the N5 comprises following components:
25-35 wt% of ginsenoside Rk3;
65-75 wt% of ginsenoside Rh4;
wherein the N6 comprises following components:
18-20 wt% of 20(S)-ginsenoside Rg3;
37-43 wt% of 20(R)-ginsenoside Rg3;
5-7 wt% of ginsenoside Rk1;
9-13 wt% of ginsenoside Rg5;
4-6 wt% of 20(S)-ginsenoside Rh2;
13-17 wt% of 20(R)-ginsenoside Rh2;
0.2-0.8 wt% of ginsenoside Rk2;
0.7-1.2 wt% of ginsenoside Rh3;
and a sum of the components of each of N1, N2, N4, N5, N6, and N7 is 100%.

3. The ginsenoside composition acting on hair according to claim 1, wherein the ginsenoside composition comprises at least one of N1, N2, N4, and at least one of N4, N5, N6.

4. A daily chemical acting on hair, comprising the ginsenoside composition according to claim 1-3;
wherein a concentration of the ginsenoside composition is 0.05-10,000 µg/mL; preferably, the concentration of the ginsenoside composition is 1-10,000 µg/mL.

5. The daily chemical acting on hair according to claim 4, wherein the concentration of the ginsenoside composition is 10-10,000 µg/mL.

6. The daily chemical acting on hair according to claim 4, wherein the daily chemical is shampoo, a hair conditioner, lyophilized powder, an essence, hair spray, hair cream, hair lotion.

7. Use of a ginsenoside composition as an anti-hair loss active ingredient in daily chemical, wherein the ginsenoside composition is N1, N2, or N4;
wherein the N1 comprises following components:
12-28 wt% of ginsenoside Rg1;
10-16 wt% of ginsenoside Re;
12-28 wt% of ginsenoside Rb1;
13-22 wt% of ginsenoside Rc;
13-22 wt% of ginsenoside Rb2;
7-13 wt% of ginsenoside Rd;
wherein the N2 comprises following components:
30-50 wt% of ginsenoside Rg1;
48-72 wt% of ginsenoside Re;
wherein the N4 comprises following components:
8-16 wt% of 20(S)-ginsenoside Rg2;
7-15 wt% of 20(R)-ginsenoside Rg2;
17-25 wt% of 20(S)-ginsenoside Rh1;
16-23 wt% of 20(R)-ginsenoside Rh1;
5-12 wt% of ginsenoside Rk3;
16-26 wt% of ginsenoside Rh4;
and a sum of the components of each of N1, N2, and N4 is 100%.

8. The use according to claim 7, wherein the N1 comprises following components:
18-22 wt% of ginsenoside Rg1;
12-15 wt% of ginsenoside Re;
18-22 wt% of ginsenoside Rb1;
15-19 wt% of ginsenoside Rc;
16-20 wt% of ginsenoside Rb2;
9-11 wt% of ginsenoside Rd;
wherein the N2 comprises following components:
35-45 wt% of ginsenoside Rg1;
55-65 wt% of ginsenoside Re;
wherein the N4 comprises following components:
10-13 wt% of 20(S)-ginsenoside Rg2;
8-13 wt% of 20(R)-ginsenoside Rg2;
18-23 wt% of 20(S)-ginsenoside Rh1;
17-21 wt% of 20(R)-ginsenoside Rh1;
6-10 wt% of ginsenoside Rk3;
18-22 wt% of ginsenoside Rh4;
and a sum of the components of each of N1, N2, and N4 is 100%.

9. Use of a ginsenoside composition as an anti-hair loss active ingredient in daily chemical, wherein the ginsenoside composition is N4, N5, or N6;
wherein the N4 comprises following components:
8-16 wt% of 20(S)-ginsenoside Rg2;
7-15 wt% of 20(R)-ginsenoside Rg2;
17-25 wt% of 20(S)-ginsenoside Rh1;
16-23 wt% of 20(R)-ginsenoside Rh1;
5-12 wt% of ginsenoside Rk3;
16-26 wt% of ginsenoside Rh4;
wherein the N5 comprises following components:
20-40 wt% of ginsenoside Rk3;
60-80 wt% of ginsenoside Rh4;
wherein the N6 comprises following components:
16-21 wt% of 20(S)-ginsenoside Rg3;
32-50 wt% of 20(R)-ginsenoside Rg3;
4-8 wt% of ginsenoside Rk1;
8-15 wt% of ginsenoside Rg5;
3-7 wt% of 20(S)-ginsenoside Rh2;
11-19 wt% of 20(R)-ginsenoside Rh2;
0.1-1 wt% of ginsenoside Rk2;
0.5-1.5 wt% of ginsenoside Rh3;
and a sum of the components of each of N4, N5, and N6 is 100%.

10. The use according to claim 9, wherein the N4 comprises following components:
10-13 wt% of 20(S)-ginsenoside Rg2;
8-13 wt% of 20(R)-ginsenoside Rg2;
18-23 wt% of 20(S)-ginsenoside Rh1;
17-21 wt% of 20(R)-ginsenoside Rh1;
6-10 wt% of ginsenoside Rk3;
18-22 wt% of ginsenoside Rh4;
wherein the N5 comprises following components:
25-35 wt% of ginsenoside Rk3;
65-75 wt% of ginsenoside Rh4;
wherein the N6 comprises following components:
18-20 wt% of 20(S)-ginsenoside Rg3;
37-43 wt% of 20(R)-ginsenoside Rg3;
5-7 wt% of ginsenoside Rk1;
9-13 wt% of ginsenoside Rg5;
4-6 wt% of 20(S)-ginsenoside Rh2;
13-17 wt% of 20(R)-ginsenoside Rh2;
0.2-0.8 wt% of ginsenoside Rk2;
0.7-1.2 wt% of ginsenoside Rh3;
and a sum of the components of each of N4, N5, and N6 is 100%.
